(19) Europäisches Patentamt
European Patent Office
Office européen des brevets

(11) EP 0 542 099 B1

(12) **EUROPÄISCHE PATENTSCHRIFT**

(45) Veröffentlichungstag und Bekanntmachung des Hinweises auf die Patenterteilung:
**03.06.1998 Patentblatt 1998/23**

(51) Int. Cl.$^6$: **C07D 233/38**

(21) Anmeldenummer: **92118796.9**

(22) Anmeldetag: **03.11.1992**

(54) **Verfahren zur Herstellung optisch aktiver 1,3-Imidazolidin-4-one**

Process for preparation of optically active 1,3-imidazolidin-4-ones

Procédé pour la préparation des imidazolidin-4-ones 1,3

(84) Benannte Vertragsstaaten:
**AT BE CH DE DK ES FR GB GR IE IT LI LU NL PT SE**

(30) Priorität: **12.11.1991 DE 4137186**
**15.11.1991 DE 4137663**

(43) Veröffentlichungstag der Anmeldung:
**19.05.1993 Patentblatt 1993/20**

(73) Patentinhaber: **DEGUSSA AG**
**60311 Frankfurt (DE)**

(72) Erfinder:
- **Seebach, Dieter, Prof. Dr.**
  **CH-8044 Zürich (CH)**
- **Drauz, Karlheinz, Dr.**
  **W-6463 Freigericht 1 (DE)**
- **Kottenhahn, Matthias, Dr.**
  **W-6450 Hanau 7 (DE)**
- **Lotter, Hermann, Dr.**
  **W-6452 Heinburg 2 (DE)**
- **Schwarm, Michael, Dr.**
  **W-8755 Alzenau (DE)**

(56) Entgegenhaltungen:
**EP-A- 0 137 351** **EP-A- 0 237 630**

- **ANGEWANDTE CHEMIE Bd. 98, Nr. 4, 1986, WEINHEIM Seiten 363 - 364 ROBERT FITZI ET AL. 'Enantiomerentrennung von (R,S)-2-(tert-Butyl)-3-methyl-4-imidazolidinon, einem chiralen Baustein für die Aminosäuresynthese'**
- **TETRAHEDRON Bd. 44, Nr. 17, 1988, GREAT BRITAIN Seiten 5277 - 5292 ROBERT FITZI ET AL. 'Resolution and use in alpha-amino acid synthesis of imidazolidinone glycine derivatives'**
- **LIEBIGS ANNALEN DER CHEMIE 1989, WEINHEIM Seiten 1215 - 1232 DIETER SEEBACH ET AL. 'Synthesis of Nonproteinogenic (R)- or (S)-Amino acids Analogues of Phenylalanine,...'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 542 099 B1

Printed by Xerox (UK) Business Services
2.16.3/3.4

## Beschreibung

Die Erfindung betrifft ein Verfahren zur Herstellung enantiomerenreiner 1,3-Imidazolidin-4-one der allgemeinen Formel

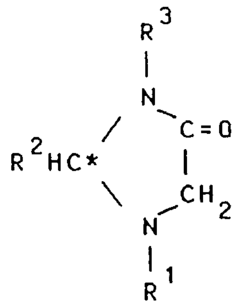

(I)

in der * ein Asymmetriezentrum ist, auf das sich die Enantiomerenreinheit bezieht, $R^1$ ist H oder eine im sauren abspaltbare Gruppe, beispielsweise Boc-, Z- oder Bz-, $R^2$ ist i-Propyl- oder t-Butyl-, und $R^3$ ist ein Methyl-, Ethyl-, Propyl- oder Benzylrest, durch Racematspaltung eines racemischen 1,3-Imidazolidin-4-ons der allgemeinen Formel

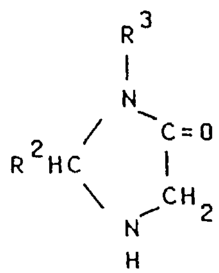

(II)

in der $R^2$ und $R^3$ die bereits angegebene Bedeutung haben, und gegebenenfalls anschließende Substitution des H am N des erhaltenen Enantiomeren zur Einführung der im sauren abspaltbaren Gruppe.

Solche Verfahren sind aus den DE-en 33 34 855 (EP-A-0 137 351) und 36 04 591 A1 (EP-A-0 237 630) sowie aus D. Seebach et al. Helv. Chim. Acta 68, 135, 949 (1985), D. Seebach et al. Angew. Chem. 98, 363 (1986) und D. Seebach et al. Tetrahedron 44, 5277 (1988) bekannt.

Die 1,3-Imidazolidin-4-one stellen eine interessante Gruppe von Verbindungen dar, die wertvolle Edukte für die Darstellung verzweigter oder unverzweigter, proteinogener oder nicht proteinogener $\alpha$-Aminosäuren durch ein- oder zweimalige diastereoselektive Alkylierung und anschließende Ringspaltung sind. Diese Aminosäuren können in Peptide eingebaut oder in Aminosäuregemischen, wie Infusionslösungen, verwendet werden. Da diese Verbindungen oder Zubereitungen als pharmazeutische Produkte Verwendung finden, besteht der Wunsch, schon die 1,3-Imidazolidin-4-one möglichst kostengünstig und insbesondere in möglichst hoher Reinheit darzustellen. Gerade dies bereitete bei den bekannten Verfahren Schwierigkeiten, da hier eine erhebliche Anzahl von einzelnen Schritten notwendig ist, bei denen öfters auch ölige statt kristalline Zwischenprodukte auftreten, so daß sich diese Verfahren nicht oder nur bedingt für die Darstellung der 1,3-Imidazolidin-4-one im größeren Maßstab eignen.

Aufgabe der vorliegenden Erfindung ist es daher, Verfahren zur Verfügung zu stellen, mit denen möglichst kostengünstig möglichst saubere und enantiomerenreine Produkte erhältlich sein sollen. Dabei soll auf aufwendige Verfahrensschritte wie z. B. Extraktionen und problematische Lösungsmittel, wie z. B. Di- oder Trichlormethan zumindest weitgehend verzichtet werden können. Je nach Kosten der Ausgangsverbindungen soll eine Optimierung hinsichtlich der notwendigen Verfahrensschritte, der Ausbeute und/oder der Reinheit der erhaltenen die so 1,3-Imidazolidin-4-one möglich sein. Außerdem sollen die so erhaltenen enantiomerenreinen 1,3-Imidazolidin-4-one zur Herstellung von enantiomerenreinen $\alpha$-Aminosäuren verwendbar sein.

Diese Aufgabe wird mit einem Verfahren zur Herstellung enantiomerenreiner 1,3-Imidazolidin-4-one der allgemeinen Formel

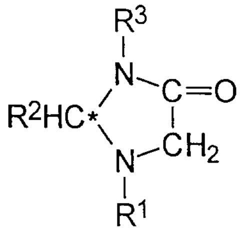

(I)

in der * ein Asymmetriezentrum ist, auf das sich die Enantiomerenreinheit bezieht, $R^1$ eine im sauren abspaltbare Gruppe oder H, $R^2$ i-Propyl oder t-Butyl und $R^3$ Methyl, Ethyl, Propyl oder Benzyl ist, durch Racematspaltung eines racemischen 1,3-Imidazolidin-4-ons der allgemeinen Formel

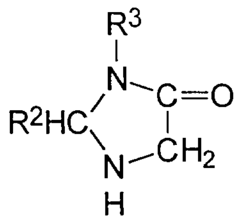

(II)

in der $R^2$ und $R^3$ die bereits angegebene Bedeutung haben, und gegebenenfalls anschließende Substitution des H am N des erhaltenen Enantiomeren zur Einführung der im sauren abspaltbaren Gruppe, gelöst, wobei sich das Verfahren dadurch auszeichnet, daß man das 1,3-Imidazolidin-4-on der allgemeinen Formel II vor der Racematspaltung als Salz isoliert und als Salz mit Aceton als Lösungsmittel und mit der stöchiometrischen Menge einer wasserfreien Base oder einer konzentrierten wässrigen Lösung einer Base behandelt, wobei das 1,3-Imidazolidin-4-on der allgemeinen Formel II löslich ist und ein Salz aus einem Kation der Base und einem Anion des Salzes des 1,3-Imidazolidin-4-ons praktisch quantitativ ausfällt, daß man das ausgefällte Salz abtrennt und das gelöste 1,3-Imidazolidin-4-on der Racemattrennung mit 0,5 - 0,8 eq eines Enantiomeren der Mandelsäure, bezogen auf das 1,3-Imidazolidin-4-on der allgemeinen Formel II, zuführt und daß man bei der Racemattrennung das gewünschte Enantiomere der allgemeinen Formel I ($R^1$ = H) durch Zusatz von 0,5 - 0,8 eq eines Enantiomeren der Mandelsäure, bezogen auf das 1,3-Imidazolidin-4-on der allgemeinen Formel II, als Salz auskristallisiert, dieses Salz mit Aceton als Lösungsmittel und einer Base ausgewählt aus der Gruppe konzentrierte Natronlauge, konzentrierte Kalilauge oder Aminbase versetzt, wobei das 1,3-Imidazolidin-4-on der allgemeinen Formel I ($R^1$ = H) löslich ist und ein Salz aus einem Kation der Base und dem Enantiomeren der Mandelsäure ausfällt, daß man das ausgefällte Salz abtrennt und das enantiomerenreine Produkt ggf. substituiert.

Das neue Verfahren erlaubt gegenüber den bisher bekannten eine neuartige Reaktionsführung, mit der, verglichen mit den bisher bekannten Verfahren, eine einfachere, billigere, schnellere und mit weniger Abfall verbundene Herstellung von Verbindungen des Typs I, die für die enantioselektive Synthese von α-Aminosäuren genutzt werden können, möglich wird. Andererseits kann für die neuen Verfahren auf aus der genannten Literatur bekannte Zwischenschritte bzw. Darstellung von Edukten zurückgegriffen werden.

Grundsätzlich kann für die Edukte der erfindungsgemäßen Verfahren von einem Alkylester der Aminosäure Glycin, vorzugsweise dem Methylester in Form seines Hydrochlorids, ausgegangen werden, der in einem niederen Alkohol mit einem niederen Monoalkylamin in bekannter Weise zum entsprechenden Glycinalkylamid-Hydrochlorid III umgesetzt wird.

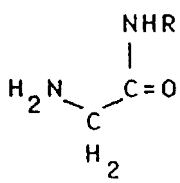

$\cdot HCl$ (III)

Dieses wird in ebenfalls bekannter Weise mit einem Aldehyd in Gegenwart einer Aminbase, wie z. B. Triethylamin, durch Kochen mit einem geeigneten Lösungsmittel, z. B. Methyl-t-butylether oder einem chlorierten oder nicht chlorierten Kohlenwasserstoff, am Wasserscheider zur Schiff'schen Base IV kondensiert, wobei über den Aldehyd der Rest $R^2$ eingeführt wird.

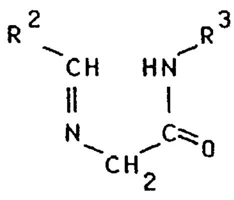

(IV)

Das hierbei entstehende Aminhydrochlorid wird filtriert und das die Schiff'sche Base enthaltende Filtrat in einem geeigneten Lösungsmittel, wie einem Ether, einem chlorierten oder nicht chlorierten Kohlenwasserstoff oder einem Alkohol, vorzugsweise Methanol, Ethanol oder Isopropanol, mit einer geeigneten, meist starken Säure, vorzugsweise wasserfreiem HCl-Gas, cyclisiert. Vorteilhaft wird nun das gebildete Salz des racemischen 1,3-Imidazolidin-4-ons II durch Abkühlen ausgefällt und durch Filtration abgetrennt, wofür Art und Menge des Lösungsmittels geeignet abzustimmen sind. Hier und im folgenden wird unter dem Begriff "Salz des 1,3-Imidazolidin-4-ons" eine Additionsverbindung mit einer Säure, wie z. B. HCl oder Mandelsäure verstanden. Durch diese Kristallisation kann II in hohen Ausbeuten und vor allem sehr hoher Reinheit erhalten werden, wobei sich herausstellte, daß das eine Bedingung für das erfolgreiche Gelingen der nachfolgenden Racematspaltung ist. Außerdem kann II als Salz im Gegensatz zu der freien Base für längere Zeit ohne Zersetzung gelagert werden. Gegenüber den bisherigen Verfahren (DE 36 04 591 A1 und Tetrahedron) wird hierbei ein fein kristallines Zwischenprodukt isoliert, wodurch die Qualität und z. T. auch die Ausbeute des gewünschten Produktes ausgesprochen günstig beeinflußt wird.

In prinzipiell bekannter Weise kann das racemische 1,3-Imidazolidin-4-on II anschließend aus seinem Salz durch Umsetzen mit einer Base auf einfache Weise freigesetzt werden. Bisher wurde statt dessen, wenn intermediär ein solches Salz erhalten wurde, dieses mit einer wässrigen Base versetzt und
die freie Base mit Methylenchlorid extrahiert,
welches für die folgende Racematspaltung wieder durch Destillation entfernt und durch Aceton ersetzt werden mußte.

Erfindungsgemäß kann vorteilhaft jetzt auf die Extraktion verzichtet werden, indem man das Salz in Aceton suspendiert und mit der stöchiometrischen Menge einer wasserfreien Base, z. B. einer Aminbase, vorzugsweise Triethylamin, oder einer konzentrierten wässrigen Lösung einer Base, wie Natrium- oder Kaliumhydroxid, versetzt. In dem entstehenden System ist das freie 1,3-Imidazolidin-4-on II löslich, während das Salz aus dem Kation der Base und dem Anion des Salzes von II praktisch quantitativ ausfällt und durch Filtration abgetrennt werden kann. Das aus der Verwendung der wässrigen Lösung der Base noch im Aceton vorhandene Wasser beeinflußt die folgende Racematspaltung nicht negativ.

Die Racematspaltung kann wieder in prinzipiell bekannter Weise durch Zusatz einer optisch aktiven Mandelsäure erfolgen. Hierbei hat sich nun erfindungsgemäß herausgestellt, daß es ausreichend ist, wenn die optisch aktive Säure in nur 0,5 - 0,8 Äquivalenten, bezogen auf das racemische 1,3-Imidazolidin-4-on der allgemeinen Formel II eingesetzt wird, wohingegen bisher ein Äquivalent oder mehr eingesetzt wurde.

Auf diese Weise kann bis zu 50 % und mehr dieser teuren chiralen Hilfsverbindung eingespart werden.
Beim Abkühlen der Lösung kristallisiert dann eines der diastereomeren Salze aus dem Kation von I und dem Anion der chiralen Säure aus und kann durch Filtration abgetrennt werden. Die optische Reinheit ist dabei im allgemeinen so hoch, daß auf eine Umkristallisation verzichtet werden kann.

Das Salz kann nun erfindungsgemäß analog der schon oben beschriebenen Vorgehensweise in Aceton als Lösungsmittel suspendiert werden, in dem das freie, optisch aktive 1,3-Imidazolidin-4-on der allgemeinen Formel I ($R^1$ = H) löslich und ein Salz aus dem Kation einer zugesetzten Base, wie z. B. Natron-oder Kalilauge oder einer Aminbase wie Triethylamin, und dem Anion der optisch aktiven Säure ausfällt. Nach beendeter Reaktion wird dieses Salz durch Filtration abgetrennt, und das gelöste 1,3-Imidazolidin-4-on der Formel I ($R^1$ = H) kann zur Einführung der im sauren abspaltbaren Gruppe in prinzipiell bekannter Weise mit einem Acylierungsmittel wie einem Säurechlorid V, einem Säureanhydrid VI, einem Kohlensäureesterchlorid VII, oder einem Pyrokohlensäurediester VIII umgesetzt werden. $R^1$ bedeutet eine bei dem jeweiligen Reagens spezifizierte, im sauren abspaltbare Gruppe, beispielsweise Boc-, Z- oder Bz-, und $R^4$ eine verzweigte oder nicht verzweigte, substituierte oder unsubstituierte Alkyl- (z. B. t-Butyl) oder Arylgruppe (z. B. Phenyl oder Benzyl).

$R^1 - X$

$R^1 = R^4 - C(=O)-, \quad X = Cl$ (V)

$R^1 = R^4 - C(=O)-, \quad X = -O-R^1$ (VI)

$R^1 = R^4 - O - C(=O)-, \quad X = Cl$ (VII)

$R^1 = R^4 - O - C(=O)-, \quad X = -O-R^1$ (VIII)

Das bei Verwendung von Alkalilauge zur Freisetzung des 1,3-Imidazolidin-4-ons I in Aceton verbliebene Wasser stört hier überraschenderweise die Acylierung von I mit V, VI, VII oder VIII nicht. Der besondere Vorteil dieses nicht-extraktiven Verfahrens besteht darin, daß auf den sonst fast unumgänglichen Einsatz eines chlorierten Kohlenwasserstoffes, insbesondere Chloroform oder Methylenchlorid, zur Extraktion des freien, am N-1 unsubstituierten, enantiomerenreinen 1,3-Imidazolidin-4-ons I aus wässriger Lösung verzichtet werden kann, was sowohl aus Umweltschutz-, Kosten- wie aus Gesundheitsgründen, insbesondere bei großtechnischen Verfahren, erstrebenswert ist und den verfahrenstechnischen Aufwand erheblich reduziert.

Ein weiterer Vorteil dieses Verfahrens ist, daß das abfiltrierte Salz aus dem Kation der zugesetzten Base und dem Anion der chiralen Säure in Form einer konzentrierten wäßrigen Lösung direkt wieder für weitere Racematspaltungen eingesetzt werden kann, so daß spezielle Aufarbeitungs- oder Reinigungsoperationen zur Rückgewinnung der wertvollen optisch aktiven saure aus diesem Reaktionsschritt überflüssig werden.

Nach Durchführung der Substitution ist oder wird erfindungsgemäß das Reaktionsprodukt in einem Lösungsmittel aufgenommen, das mit Wasser nicht frei mischbar ist und aus welchem das Reaktionsprodukt kristallisiert werden kann. Besonders geeignet sind hier Kohlenwasserstoffe wie beispielsweise n-Hexan oder Cyclohexan, aber auch Petrolether verschiedener Siedebereiche. Anschließend wird die Lösung des Produktes mit Wasser gewaschen. Damit werden die bei der Acylierung entstehenden Produkte wie HCl, $R^4COOH$ oder $R^4OH$, welche sonst die nachfolgende Kristallisation des Produktes stören und damit die Ausbeute vermindern können, zum größten Teil abgetrennt.

Alternativ oder zusätzlich zu dem letzteren Schritt kann ein Teil des Lösungsmittels abdestilliert werden, wobei die Destillation vorzugsweise nach dem vorgenannten Schritt durchgeführt wird. Hierbei werden die o. g. Nebenprodukte der Acylierung noch weiter, d. h. praktisch vollständig abgetrennt.

Bei dieser Verfahrensweise kann vorteilhaft das Imidazolidinon der Formel II nach der Racematspaltung in einem wässrigen System mit einem mit Wasser mischbaren organischen Lösungsmittel (z. B. Aceton) versetzt werden. Anschließend führt man in diesem System die im sauren abspaltbare Gruppe ein, trennt das organische Lösungsmittel ab und nimmt das Produkt mit dem mit Wasser nicht frei mischbaren Lösungsmittel auf.

Zur Aufarbeitung wird das enantiomerenreine, am N-1 substituierte 1,3-Imidazolidin-4-on der allgemeinen Formel I durch Abkühlen auskristallisiert und durch Filtration abgetrennt. Durch diese Verfahrensweise gelingt es, in einem Schritt in hoher Ausbeute ein feinkristallines, sehr sauberes Produkt zu erzeugen, das im allgemeinen nicht mehr umkristallisiert werden muß. Gemäß den bisher bekannten Verfahren wurde dagegen zunächst ein Öl erhalten, das zur Kristallisation gebracht werden mußte, was insbesondere großtechnisch sehr aufwendig ist.

Die bei der Racematspaltung des racemischen 1,3-Imidazolidin-4-ons II verbliebene Mutterlauge enthält in angereicherter Form das zweite Enantiomer, welches, wenn gewünscht, nach bekannten Verfahren in reiner Form isoliert werden kann (DE 36 04 591 A1). Oft besteht jedoch nur für ein Enantiomeres eine Nachfrage, so daß es von ökonomischem wie ökologischem Vorteil ist, das in diesem Fall unerwünschte Enantiomere racemisieren zu können, um dieses aufwendig synthetisierte Zwischenprodukt besser, d. h. zu mehr als max. 50 %, auszunutzen.

Für die Racemisierung wird erfindungsgemäß das nach einem extraktiven oder nicht-extraktiven Verfahren aus der Mutterlauge der Racematspaltung oder als Salz isolierte freie 1,3-Imidazolidin-4-on in einem inerten, vorzugsweise hochsiedenden Lösungsmittel, z. B. einem Polyether, aufgenommen und durch Erhitzen racemisiert, wobei sich Temperatur und Dauer dieser Reaktion nach der Stabilität und Racemisierungsgeschwindigkeit des 1,3-Imidazolidin-4-ons in dem verwendeten Lösungsmittel richten. Im allgemeinen liegt die erforderliche Temperatur zwischen 50°C und 5 K unter dem Zersetzungspunkt der freien Base oder des Salzes. Nach Abkühlen wird diese Lösung in die Lösung einer wasserfreien Säure in einem niederen Alkohol, z. B. HCl-Gas in Methanol, getropft, aus der dann das Salz des racemischen 1,3-Imidazolidin-4-ons II auskristallisiert und durch Filtration in hoher Ausbeute isoliert werden kann.

Alternativ ist es auch möglich, zuerst ein Salz des 1,3-Imidazolidin-4-ons herzustellen, in dem ein Enantiomeres angereichert ist, welches dann in einem inerten, vorzugsweise hochsiedenden Lösungsmittel, wie z. B. einem Polyether, gelöst oder suspendiert und durch Erhitzen racemisiert wird. Temperatur und Dauer der Reaktion richten sich nach der Stabilität und der Racemisierungsgeschwindigkeit des Salzes in dem verwendeten Lösungsmittel. Im allgemeinen liegen die Temperaturen, wie beim vorhergehenden Verfahren zwischen 50°C und 5 K unter dem Zersetzungspunkt des Enantiomeren oder seines Salzes in dem Lösungsmittel. Nach Abkühlen wird das Salz des racemischen 1,3-Imidazolidin-4-ons II abfiltriert und, falls erforderlich, durch Umkristallisation gereinigt. Die geschilderten Verfahren haben gegenüber der bisher beschriebenen Racemisierung durch Erhitzen der freien Base in Substanz (D. Seebach, Ang. Chem. 102, 1363 (1990)) den Vorteil, daß sie nahezu zersetzungsfrei ablaufen, dadurch gute Ausbeuten liefern und vor allem auch in technischem Maßstab problemlos durchführbar sind. Insbesondere ist hierbei überraschend, daß das Salz des 1,3-Imidazolidin-4-ons in einem Lösungsmittel suspendiert überhaupt racemisiert werden kann, da das Salz als solches (ohne Lösungsmittel) der Racemisierung nicht zugänglich ist.

Das oben beschriebene Verfahren bietet Insgesamt einen erheblich vereinfachten und technisch leicht realisierbaren Zugang zu enantiomerenreinen 1,3-Imidazolidin-4-onen der allgemeinen Formel I, wobei weniger Zeit und teure Ausgangsverbindungen als bisher benötigt und die unvermeidliche Menge an Abfallstoffen drastisch reduziert wurde. Insbesondere wird auf die Verwendung halogenierter Kohlenwasserstoffe völlig verzichtet.
Durch die erfindungsgemäßen Verfahrensschritte wurde das seit vielen Jahren bekannte Verfahren zur Herstellung der Verbindung der allgemeinen Formel I für technische Produktionen wirtschaftlich.

Die erhaltenen Imidazolidinone dienen vorteilhaft zur Herstellung von enantiomerenreinen verzweigten oder unverzweigten proteinogenen oder nicht proteinogenen $\alpha$-Aminosäuren sowie diese Aminosäuren enthaltenden Peptide oder Aminosäuregemische. Hierzu werden die erhaltenen Imidazolidinone vorteilhaft in bekannter Weise diastereoselektiv alkyliert. Aus dem alkylierten Produkt kann dann in bekannter Weise durch Ringspaltung die entsprechende $\alpha$-Aminosäure erhalten werden, die dann weiter umgesetzt - z. B. in ein Peptid eingebaut - oder in sonstiger Weise - z. B. in einem Aminosäuregemisch - eingesetzt werden kann. (W. Müller, D. A. Lowe, H. Neijt, S. Urwyler, P. L. Herrling, D. Blaser, D. Seebach, Helv. Chim. Acta 1992, 75, 855; D. Seebach, E. Dziadulewicz, L. Behrendt, S. Cantoreggi, R. Fitzi, Liebigs Ann. Chem. 1989, 1215; R. Fitzi, D. Seebach, Tetrahedron 1988, 44, 5277.)

Das erfindungsgemäße Verfahren wird im folgenden anhand von Ausführungsbeispielen näher beschrieben.

Beispiel 1 (Ausgangsverbindung)

Herstellung von (R,S)-2-(t-Butyl)-3-methyl-1,3-imidazolidin-4-on-hydrochlorid

In eine Lösung von 77,5 g (2,5 mol) Monomethylamin in 618 ml Methanol wurden bei Raumtemperatur 125,6 g (1,0 mol) Glycinmethylester-hydrochlorid fest eingetragen und die entstandene Lösung über Nacht bei Raumtemperatur gerührt. Anschließend wurde ein Großteil des Methanols bei leichtem Vakuum abdestilliert und nach Zusatz von 250 ml Cyclohexan das verbliebene Methanol azeotrop abgeschieden. Der Rückstand wurde mit 300 ml Methyl-t-butylether, 167 ml (1,5 mol) Pivalaldehyd und 152 g (1,5 mol) Triethylamin versetzt und 8,5 h am Wasserabscheider unter Rückfluß erhitzt, wobei 24,5 ml Wasser abgetrennt wurden. Danach wurde der Ether abdestilliert und die als Rückstand verbleibende Schiff'sche Base in 100 ml Ethanol aufgenommen.

Zu dieser Lösung wurden dann unter Eiskühlung innerhalb von 45 min 332 g einer 13,2 %igen Lösung von HCl-Gas (1,2 mol) in Ethanol getropft. Nach 15 min schieden sich feine, weiße Kristalle ab. Zur Vervollständigung der Fällung wurden bei 175 mbar noch 80 ml Ethanol abdestilliert und 400 ml Methyl-t-butylether zugetropft. Nach 1,5 h Rühren im Eisbad wurde der Feststoff abgenutscht, mit 150 ml Methyl-t-butylether gewaschen und im Vakuumtrockenschrank bei 35°C getrocknet.

Ausbeute: 123,0 g (63,8 %)
Smp.: 185,0 - 186,5°C

| | | | | |
|---|---|---|---|---|
| $C_8H_{17}ClN_2O$ ber. | C 49,86 | H 8,89 | N 14,58 | Cl 18,40 |
| MG: 192,7 gef. | C 49,80 | H 9,11 | N 14,21 | Cl 18,36 |

$^1$H-NMR (500 MHz, $d^6$-DMSO): 1,1 ppm (s, 9 H, C(C$\underline{H}_3$)$_3$), 2,9 (s, 3 H, NC$\underline{H}_3$), 3,8 (AB-System, 2 H, C$\underline{H}_2$), 4,7 (s, 1 H, NC$\underline{H}$N), 10,4 (br s, 2 H, N$\underline{H}_2^+$).

Beispiel 2

Enantiomerentrennung von (R,S)-2-(t-Butyl)-3-methyl-1,3-imidazolidin-4-on mit 0,5 Äquivalenten Mandelsäure

Eine Lösung von 1,56 g (0,010 mol) (R,S)-2-(t-Butyl)-3-methyl-1,3-imidazolidin-4-on in 5 ml Aceton wurde zu einer Lösung von 0,76 g (0,005 mol) (R)-Mandelsäure in 5 ml Aceton gegeben, wobei sich spontan ein Kristallbrei bildete. Durch Erwärmen auf ca. 50°C wurde eine klare, gelbe Lösung erhalten, aus der beim langsamen Abkühlen auf 5°C farbloses, feinkristallines (R)-2-(t-Butyl)-3-methyl-1,3-imidazolidin-4-on-(R)-mandelat ausfiel, das abfiltriert, mit fünfmal 1 ml kaltem Aceton gewaschen und im Vakuumtrockenschrank bei ca. 50°C getrocknet wurde.

Ausbeute: 0,99 g (64,2 %)
Optische Reinheit (GC,TFA-Derivat, Chirasil-Val): 99,15 % (R)-I ($R^1$ = H)

Beispiel 3

Darstellung von (S)-1-(t-Butoxycarbonyl)-2-(t-butyl)-3-methyl-1,3-imidazolidin-4-on

Zu einer Suspension von 308,3 g (1,0 mol) (R)-2-(t-Butyl)-3-methyl-1,3-imidazolidin-4-on-(R)-mandelat in 1,2 l Aceton wurden unter Rühren 80,0 g (1,0 mol) 50 %ige Natronlauge getropft. Nach 1 h Reaktionszeit bei Raumtemperatur wurde das unlösliche Natrium-(R)-mandelat abfiltriert, mit 150 ml Aceton gewaschen und im Vakuumtrockenschrank bei ca. 50°C getrocknet.

Ausbeute: 159,6 g (91,6 %)
$[\alpha]_D^{20}$ : -143.5° (c = 2, 2N HCl)

Das Filtrat wurde innerhalb von 45 min unter Eiskühlung mit 229,0 g (1,05 mol) Di-(t-butyl)-pyrocarbonat ("Diboc") versetzt. Nach kurzer Nachrührzeit wurde das Aceton abdestilliert, der Rückstand in 300 ml n-Hexan aufgenommen und mit 150 ml Wasser gewaschen. Anschließend wurde das Hexan abdestilliert, der Rückstand erneut in 500 ml n-Hexan aufgenommen und über Nacht auf 5°C gekühlt. Das auskristallisierte (S)-1-(t-Butoxycarbonyl)-2-(t-butyl)-3-methyl-1,3-imidazolidin-4-on wurde
abfiltriert, mit 150 ml kaltem n-Hexan gewaschen und im Vakuumtrockenschrank bei Raumtemperatur getrocknet. Einengen der Mutterlauge lieferte eine weitere Charge der gleichen Verbindung, die wie beschrieben isoliert wurde.

Ausbeute: 80,3 g + 95,8 g = 176,1 g (68,7 %)
$[\alpha]_D^{20}$ : 1. Charge -14,95°
2. Charge -14,70°
Analytische Daten: R. Fitzi, D. Seebach, Tetrahedron 44, 5277 - 5292, 1988.

Beispiel 4

Racemisierung von 2-(t-Butyl)-3-methyl-1,3-imidazolidin-4-on-hydrochlorid

50,0 g (0,26 mol)
2-(t-Butyl)-3-methyl-1,3-imidazolidin-4-on-hydrochlorid ($[\alpha]_D^{20}$ : +28,1°) wurden in 550 ml Diethylenglykoldimethylether ("Diglyme") suspendiert und 5,5 h auf 130°C erhitzt. Nach Abkühlen auf 20°C wurde der Feststoff abfiltriert, mit n-Hexan farblos gewaschen und bei 50°C im Vakuumtrockenschrank getrocknet.

Ausbeute: 45,5 g (91,0 %)

$[\alpha]_D^{20}$ : +0,5° (c = 2, MeOH)

Das 2-(t-Butyl)-3-methyl-1,3-imidazolidin-4-on-hydrochlorid wurde aus 160 ml Ethanol umkristallisiert und wie oben isoliert und getrocknet.

Ausbeute: 36,2 g (72,4 %)
$[\alpha]_D^{20}$ : 0 (c = 2, MeOH)
Analytische Daten: siehe Beispiel 1.

Beispiel 5

Darstellung von (S)-1-Benzoyl-2-(t-butyl)-3-methyl-1,3-imidazolidin-4-on

Zu einer Suspension von 93,5 g (0,3 mol) (R)-2-(t-Butyl)-3-methyl-1,3-imidazolidin-4-on-(R)-mandelat in 360 ml Aceton wurden unter Rühren 24,0 g (0,3 mol) 50 %ige Natronlauge getropft. Nach 2 h Rühren bei Raumtemperatur wurde das unlösliche Natrium-(R)-mandelat abfiltriert, mit 150 ml Aceton gewaschen und im Vakuumtrockenschrank bei 50°C getrocknet.

Ausbeute: 47,32 g (90,6 %)
$[\alpha]_D^{20}$ : -142,1°

Das Filtrat wurde bei 0°C mit weiteren 24,0 g (0,3 mol) 50 %iger Natronlauge und anschließend tropfenweise mit 46,4 g (0,33 mol) Benzoylchlorid versetzt. Nach 1 h Rühren bei Raumtemperatur wurde das ausgefallene Kochsalz abfiltriert und mit 100 ml Aceton gewaschen. Das Filtrat wurde einrotiert, der Rückstand in 300 ml heißem Toluol gelöst, heiß mit 100 ml Wasser, 100 ml gesättigter Natriumhydrogencarbonatlösung und wieder 100 ml Wasser ausgerührt und auf etwa 150 g eingeengt. Beim Abkühlen auf -40°C entstanden farblose Kristalle, die abgesaugt, mit 2 x 10 ml Toluol gewaschen, im Vakuumtrockenschrank bei 50 bzw.75°C getrocknet und aus 120 ml Ethanol erneut umkristallisiert wurden. Nach Filtration und Trocknen wurden farblose Nadeln erhalten.

Ausbeute: 57,47 g (73,6 %)
$[\alpha]_D^{20}$ : +127,3°
Smp.: 143 - 146°C

**Patentansprüche**

**1.** Verfahren zur Herstellung enantiomerenreiner 1,3-Imidazolidin-4-one der allgemeinen Formel

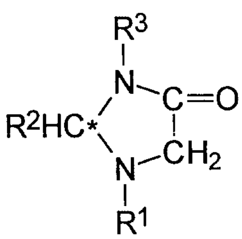

(I)

in der * ein Asymmetriezentrum ist, auf das sich die Enantiomerenreinheit bezieht, $R^1$ eine im sauren abspaltbare Gruppe oder H, $R^2$ i-Propyl oder t-Butyl und $R^3$ Methyl, Ethyl, Propyl oder Benzyl ist, durch Racematspaltung eines racemischen 1,3-Imidazolidin-4-ons der allgemeinen Formel

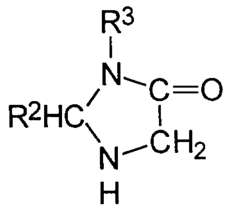

(II)

in der $R^2$ und $R^3$ die bereits angegebene Bedeutung haben, und gegebenenfalls anschließende Substitution des H am N des erhaltenen Enantiomeren zur Einführung der im sauren abspaltbaren Gruppe,
**dadurch gekennzeichnet,**
daß man das 1,3-Imidazolidin-4-on der allgemeinen Formel II vor der Racematspaltung als Salz isoliert und als Salz mit Aceton als Lösungsmittel und mit der stöchiometrischen Menge einer wasserfreien Base oder einer konzentrierten wässrigen Lösung einer Base behandelt, wobei das 1,3-Imidazolidin-4-on der allgemeinen Formel II löslich ist und ein Salz aus einem Kation der Base und einem Anion des Salzes des 1,3-Imidazolidin-4-ons praktisch quantitativ ausfällt, daß man das ausgefällte Salz abtrennt und das gelöste 1,3-Imidazolidin-4-on der Racemattrennung mit 0,5 - 0,8 eq eines Enantiomeren der Mandelsäure, bezogen auf das 1,3-Imidazolidin-4-on der allgemeinen Formel II, zuführt und daß man bei der Racemattrennung das gewünschte Enantiomere der allgemeinen Formel I ($R^1$ = H) durch Zusatz von 0,5 - 0,8 eq eines Enantiomeren der Mandelsäure, bezogen auf das 1,3-Imidazolidin-4-on der allgemeinen Formel II, als Salz auskristallisiert, dieses Salz mit Aceton als Lösungsmittel und einer Base ausgewählt aus der Gruppe konzentrierte Natronlauge, konzentrierte Kalilauge oder Aminbase versetzt, wobei das 1,3-Imidazolidin-4-on der allgemeinen Formel I ($R^1$ = H) löslich ist und ein Salz aus einem Kation der Base und dem Enantiomeren der Mandelsäure ausfällt, daß man das ausgefällte Salz abtrennt und das enantiomerenreine Produkt ggf. substituiert.

2. Verfahren nach Anspruch 1,
**dadurch gekennzeichnet,**
daß man das Imidazolidinon der Formel I ($R^1$ = H) nach der Racematspaltung in einem wässrigen System mit einem mit Wasser mischbaren organischen Lösungsmittel versetzt, die im sauren abspaltbare Gruppe einführt, das mit Wasser mischbare organische Lösungsmittel abtrennt und dann das Produkt in einem mit Wasser nicht frei mischbaren Lösungsmittel aufnimmt.

3. Verfahren gemäß Anspruch 2,
**dadurch gekennzeichnet,**
daß man das Produkt nach der Substitution in einem Lösungsmittel, das mit Wasser nicht frei mischbar ist und aus dem das Produkt umkristallisierbar ist, gelöst mit Wasser wäscht und anschließend aus dem Lösungsmittel kristallisiert.

4. Verfahren gemäß Anspruch 2,
**dadurch gekennzeichnet,**
daß man das Produkt nach der Substitution in einem Lösungsmittel gelöst, aus dem das Produkt umkristallisierbar ist, nach Abziehen eines Teils des Lösungsmittels in dem Lösungsmittel auskristallisiert.

5. Verfahren gemäß Anspruch 1,
**dadurch gekennzeichnet,**
daß man das bei der Kristallisation zurückbleibende nicht gewünschte Enantiomer als freie Base oder Salz isoliert, mit einem inerten Lösungsmittel versetzt und bei einer Temperatur zwischen 50 °C und 5 K unter dem Zersetzungspunkt der freien Base oder des Salzes racemisiert und anschließend erneut der Racemattrennung zuführt.

## Claims

1. Process for preparing enantiomerically pure 1,3-imidazolidin-4-ones of the general formula (I)

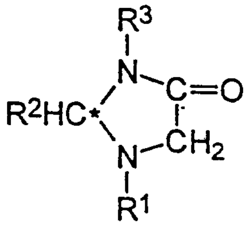

(I)

in which the * is an asymmetric centre to which the enantiomeric purity refers, $R^1$ is a group which can be eliminated in acid or H, $R^2$ is iso-propyl or t-butyl and $R^3$ is methyl, ethyl, propyl or benzyl, by racemate resolution of a racemic 1,3-imidazolidin-4-one of the general formula (II)

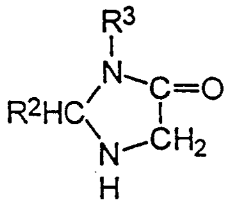

(II)

in which $R^2$ and $R^3$ are defined as above, and optionally followed by substitution of the H on the N of the enantiomer obtained to introduce the group which can be eliminated in acid,
characterised in that the 1,3-imidazolidin-4-one of the general formula II is isolated as a salt before racemate resolution and is treated as a salt with acetone as solvent and with the stoichiometric amount of an anhydrous base or a concentrated aqueous solution of a base, wherein the 1,3-imidazolidin-4-one of the general formula II is soluble and a salt formed from a cation from the base and an anion from the salt of 1,3-imidazolidin-4-one is virtually quantitatively precipitated, that the precipitated salt is isolated and 0.5 to 0.8 equivalents of an enantiomer of mandelic acid, with reference to the 1,3-imidazolidin-4-one of the general formula II is introduced to the dissolved 1,3-imidazolidin-4-one from racemate separation, and that during racemate separation the desired enantiomer of the general formula I ($R^1$ = H) is crystallised out as a salt by adding 0.5 to 0.8 equivalents of an enantiomer of mandelic acid, with respect to the 1,3-imidazolidin-4-one of the general formula II, acetone as solvent and a base selected from the group concentrated caustic soda solution, concentrated potash solution or an amine base are added to this salt, wherein the 1,3-imidazolidin-4-one of the general formula I ($R^1$ = H) is soluble and a salt comprising the cation from the base and the enantiomer of mandelic acid precipitate, that the precipitated salt is isolated and the enantiomerically pure product is optionally substituted.

2. Process according to Claim 1,
characterised in that after racemate resolution in an aqueous system, an organic solvent which is miscible with water is added to the imidazolidinone of the formula I ($R^1$ = H), the group which can be eliminated in acid is introduced, the organic solvent miscible with water is removed and then the product is taken up in a solvent which is not freely miscible with water.

3. Process according to Claim 2,
characterised in that the product after substitution is dissolved in a solvent which is not freely miscible with water and from which the product can be recrystallised, is washed with water and is then crystallised from the solvent.

4. Process according to Claim 2,
characterised in that the product after substitution is dissolved in a solvent from which the product can be recrystallised and is recrystallised after removing some of the solvent.

5. Process according to Claim 1,
characterised in that the unwanted enantiomer left behind during crystallisation is isolated as the free base or as a salt, treated with an inert solvent and racemised at a temperature between 50°C and 5K below the decomposition

point of the free base or the salt and then returned to the racemate resolution process.

**Revendications**

1. Procédé pour la préparation des énantiomères à l'état pur de 1,3-imidazolidin-4-ones répondant à la formule générale

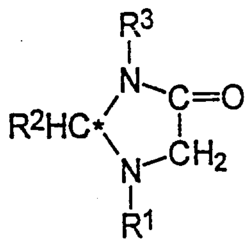

(I)

dans laquelle * représente un centre d'asymétrie auquel se rapporte la pureté des énantiomères, $R^1$ représente un groupe apte à se séparer dans des conditions acides ou encore H, $R^2$ représente un groupe i-propyle ou un groupe t-butyle, et $R^3$ représente un groupe méthyle, un groupe éthyle, un groupe propyle ou un groupe benzyle, par dédoublement d'une 1,3-imidazolidin-4-one racémique répondant à la formule générale

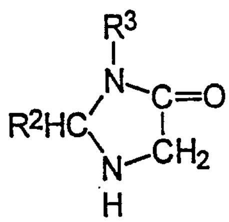

(II)

dans laquelle $R^2$ et $R^3$ possèdent la signification déjà indiquée, et éventuellement par substitution ultérieure de l'atome d'hydrogène sur l'atome d'azote de l'énantiomère obtenu pour l'introduction du groupe apte à se séparer dans des conditions acides,
caractérisé en ce qu'
on isole la 1,3-imidazolidin-4-one répondant à la formule générale II sous forme d'un sel avant le dédoublement du racémate et on la traite sous forme de sel avec de l'acétone à titre de solvant et avec la quantité stoechiométrique d'une base anhydre ou d'une solution aqueuse concentrée d'une base, dans lesquels la 1,3-imidazolidin-4-one répondant à la formule générale II est soluble et un sel constitué par un cation de la base et par un anion du sel de la 1,3-imidazolidin-4-one précipite de manière pratiquement quantitative, en ce qu on sépare le sel précipité et on achemine la 1,3-imidazolidin-4-one dissoute au dédoublement du racémate avec de 0,5 à 0,8 éq. d'un énantiomère de l'acide mandélique, rapporté à la 1,3-imidazolidin-4-one répondant à la formule générale II, et en ce qu'on sépare par cristallisation sous forme d'un sel, lors du dédoublement du racémate, l'énantiomère désiré répondant à la formule générale I ($R^1$ = H) par addition de 0,5-0,8 éq. d'un énantiomère de l'acide mandélique, rapporté à la 1,3-imidazolidin-4-one répondant à la formule générale II, on ajoute à ce sel de l'acétone à titre de solvant et une base choisie parmi le groupe comprenant de la lessive de soude concentrée, de la lessive potassique concentrée ou une base d'amine, dans lesquels la 1,3-imidazolidin-4-one répondant à la formule générale I ($R^1$ = H) est soluble et un sel constitué d'un cation de la base et de l'énantiomère de l'acide mandélique précipite, en ce qu'on sépare le sel précipité et on substitue, le cas échéant, le produit sous forme d'énantiomère à l'état pur.

2. Procédé selon la revendication 1,
caractérisé en ce que
on ajoute à l'imidazolidinone de formule I ($R^1$ = H), après le dédoublement du racémate dans un système aqueux, un solvant organique miscible à l'eau, on introduit le groupe apte à se séparer dans des conditions acides, on sépare le solvant organique miscible à l'eau, puis on reprend le produit dans un solvant qui n'est pas librement miscible à l'eau.

3. Procédé selon la revendication 2,

caractérisé en ce que
on lave avec de l'eau le produit dissous après la substitution dans un solvant qui n'est pas librement miscible à l'eau et à partir duquel le produit peut être recristallisé, puis on le cristallise hors du solvant.

4. Procédé selon la revendication 2,
caractérisé en ce que
on sépare par cristallisation dans le solvant le produit dissous après la substitution dans un solvant à partir duquel le produit peut être recristallisé, après extraction d'une partie du solvant.

5. Procédé selon la revendication 1,
caractérisé en ce que
on isole, sous forme d'une base libre ou d'un sel, l'énantiomère non désiré subsistant lors de la cristallisation, on lui ajoute un solvant inerte et on le racémise à une température entre 50°C et 5 K en dessous du point de décomposition de la base libre ou du sel et on l'achemine ensuite une nouvelle fois au dédoublement du racémate.